# EUROPEAN PATENT APPLICATION

(11) **EP 4 542 926 A1**
(43) Date of publication of application: **23.04.2025**
(21) Application number: 23843380.9
(22) Date of filing: 19.07.2023
(51) Int. Cl.: H04L 9/32, H04L 9/08, H04L 9/40

(54) **SYSTEM AND METHOD FOR DE-IDENTIFICATION OF PERSONAL INFORMATION IN MEDICAL SERVICES**

(30) Priority: 22.07.2022 KR 20220090766; 18.07.2023 KR 20230093210
(71) Applicant: Medical AI Co., Ltd., Seoul 06180 (KR)
(72) Inventor: KWON, Joonmyoung, Seoul 06180 (KR); HAN, Yoon, Seoul 06180 (KR)
(74) Representative: Patentanwaltskanzlei Matschnig & Forsthuber OG
(86) International application number: PCT/KR2023/010427
(87) International publication number: WO 2024/019534

(57) **Abstract**

Disclosed in one embodiment of the present disclosure are a system and a method by which a computing device de-identifies personal information in medical services. The method may comprise: a key server having a key for the encryption and decryption of medical data in a clinical environment; an authentication server which authenticates access information about a user client, and which issues a token for maintaining a session; a ticket server for issuing, on the basis of a request by the user client for access to the encrypting medical data, a ticket by which the user client is authorized to receive the key; and a key exchange server for transmitting, to the user client, on the basis of a request by the user client for decryption of the encrypted medical data, the key issued by the key server.

## Description

### [Technical Field]

The present disclosure relates to encryption technology in the field of medical services, and more particularly, to a system and method for safely encrypting and transmitting data in a clinical environment in a situation where medical data is transmitted in a cloud environment and enabling only users with personal information ownership rights to safely view decrypted data.

### [Background Art]

The existing de-identification methods include pseudonymization, aggregation, data deletion, data categorization, data masking, etc. Among these, methods such as data deletion, categorization, aggregation, and masking modify existing data to make the existing data unidentifiable, and therefore the original data may not be restored. Since personal information such as a patient number should be viewed in its original form when accessing services from a clinic that is the owner of the information and a user of the services, it is difficult to apply the above-described method.

Meanwhile, encryption methods include one-way encryption such as a hash function and two-way encryption from which decrypted is possible. In a symmetric key method, which is one of the two-way encryption methods, because a transmitting side and a receiving side perform encryption and decryption with the same key, safe key exchange is important.

When the entity performing encryption is a clinic and the entity performing decryption is a cloud service, if a cloud server end has an encryption key, a cloud service (or its manager) also has authority to perform decryption and becomes a handler of personal information. To prevent this, when a user has a decryption key, the security of a browser end may not be guaranteed and problems such as key loss also occur. Therefore, an algorithm is needed to perform decryption or exchange keys for decryption with the corresponding login session after a user acquires login rights.

### [Disclosure]

### [Technical Problem]

The present disclosure provides a system and method for safely encrypting and transmitting medical data in a clinical environment in a cloud environment and enabling only users with personal information ownership rights to safely view decrypted data.

However, the problems to be solved by the present disclosure are not limited to the problems described above, and other problems that are not mentioned may be clearly understood based on the description below.

### [Technical Solution]

To realize tasks described above, a system for de-identification of personal information in medical services is disclosed. The system may include: a key server that has a key for encryption and decryption of medical data in a clinical environment; an authentication server that authenticates access information about a user client and issues a token for maintaining a session; a ticket server that issues, based on a request by a user client for access to the encrypted medical data, a ticket by which the user client is authorized to receive the key; and a key exchange server that transmits, to the user client, based on a request by the user client for decryption of the encrypted medical data, the key issued by the key server.

The key server may form a communication trust interval so that only the key exchange server accesses the key server, when the server or client requesting the key is not located within the clinical environment.

When the ticket server receives, from the user client, an access request including the token issued by the authentication server, the ticket server may verify the token through communication with the authentication server, and when the token verification is completed by the authentication server, issue the ticket to the user client.

When the key exchange server receives, from the user client, the decryption request including the token issued by the authentication server, the key exchange server may transmit the token to the ticket server to request the issuance of the ticket, and the ticket server may verify the token transmitted from the key exchange server through the communication with the authentication server, and when the token verification is completed by the authentication server, transmit the ticket issued to the user client to the key exchange server based on the token.

When the key exchange server receives, from the ticket server, the ticket issued to the user client based on the token, the key exchange server may receive a key for decryption of the encrypted medical data through the communication with the key server, encrypt the key received through the communication with the key server with the ticket issuing the token to the user client, and transmit the key encrypted with the ticket to the user client.

The user client may decrypt the key received from the key exchange server using the ticket issued through the ticket server, and decrypt the medical data using the key decrypted through the ticket.

The user client may not store the key received from the key exchange server, and use the key for decryption of the medical data and then discards the key.

The authentication server, the ticket server, or the key exchange server may perform validity verification on a timestamp generated during the communication with the user client or communication between servers, and discard the received information corresponding to the timestamp whose valid time has exceeded when the timestamp is determined to have exceeded its valid time through the validity verification.

To realize tasks described above, a method of de-identification of personal information in medical services is disclosed. The method may include: issuing, by a ticket server, a ticket based on a request by a user client for access to encrypted medical data; transmitting, by a key exchange server, a key issued from a key server in a clinic to the user client based on a request by the user client for decryption of the encrypted medical data; and decrypting, by the user client, the key transmitted from the key exchange server using the issued ticket.

### [Advantageous Effects]

According to the method of the present disclosure, by safely managing the key used for the data encryption within the clinic, it is possible to prevent the personal information from being used even when the information is leaked due to the hacking of the cloud services.

In addition, since the ticket is not issued without the token issued upon the user authentication and since the user or manager cannot arbitrarily acquire the key and arbitrarily steal the key from any location on the cloud when the ticket is not the valid ticket, it is possible to significantly improve the security in the medical cloud services environment.

### [Description of Drawings]

FIG. 1 is a block diagram illustrating a system for de-identification of personal information in medical services according to an embodiment of the present disclosure.
FIG. 2 is a block diagram illustrating an encryption process according to an embodiment of the present disclosure.
FIG. 3 is a block diagram illustrating a decryption process according to an embodiment of the present disclosure.
FIG. 4 is a flowchart illustrating a method of de-identification of personal information in medical services according to an embodiment of the present disclosure.
FIG. 5 is a block diagram of a computing device constituting the system according to an embodiment of the present disclosure.

### [Best Mode]

Hereinafter, embodiments of the present disclosure will be described in detail with reference to the attached drawings so that those skilled in the art can easily implement the present disclosure. The embodiments presented in the present disclosure are provided so that those skilled in the art may utilize or implement contents of the present disclosure. Accordingly, various modifications to the embodiments of the present disclosure will be apparent to those skilled in the art. That is, the present disclosure may be implemented in various different forms, and is not limited to embodiments described below.

Throughout the specification of the present disclosure, the same or similar drawing symbols refer to the same or similar components. In addition, in order to clearly describe the present disclosure, drawing symbols of parts that are not related to the description of the present disclosure may be omitted in the drawings.

The term "or" used in the present disclosure is intended to mean an inclusive "or", not an exclusive "or." That is, unless otherwise specified in the present disclosure or the meaning is clear from the context, "X uses A or B" is intended to mean one of the natural inclusive substitutions. For example, unless otherwise specified in the present disclosure or the meaning is clear from the context, "X utilizes A or B" may be construed as one of X utilizes A, X utilizes B, or X utilizes both A and B.

The term "and/or" used in the present disclosure should be understood to refer to and include all possible combinations of one or more of the related concepts listed.

The terms "include" and/or "including" used in the present disclosure should be understood to mean that specific features and/or components are present. However, the terms "include" and/or "including" should be understood as not excluding the presence or addition of one or more other features, components and/or groups thereof.

In addition, unless otherwise specified in the present disclosure or the context is clear to indicate a singular form, the singular form should generally be construed to include "one or more."

The term "Nth (N is a natural number)" used in the present disclosure may be understood as expressions used to mutually distinguish components of the present disclosure according to a predetermined standard such as a functional perspective, a structural perspective, or convenience of description. For example, components performing different functional roles in the present disclosure may be distinguished as a first component or a second component. However, components that are substantially the same within the technical idea of the present disclosure but should be distinguished for convenience of description may also be distinguished as the first component or the second component.

The term "acquisition" used in the present disclosure may be understood to mean not only receiving data through a wired or wireless communication network with an external device or system, but also generating data in an on-device form.

Meanwhile, the term "module" or "unit" used in the present disclosure may be understood as a term referring to an independent functional unit that processes computing resources, such as a computer-related entity, firmware, software or a part thereof, hardware or a part thereof, a combination of software and hardware. In this case, the "module" or "unit" may be a unit composed of a single element, or may be a unit expressed as a combination or set of multiple elements. For example, as a narrow concept, the "module" or "unit" may refer to hardware elements of a computing device or a set thereof, an application program that performs a specific function of software, a processing process implemented through software execution, a set of instructions for program execution, etc. In addition, as a broad concept, the "module" or "unit" may refer to a computing device itself that constitutes a system, an application that is executed on the computing device, etc. However, the above-described concept is only an example, and the concept of "module" or "unit" may be variously defined within a category understandable to those skilled in the art based on the contents of the present disclosure.

The term "model" used in the present disclosure may be understood as a system implemented using mathematical concepts and language to solve a specific problem, a set of software units to solve a specific problem, or an abstraction model of a processing process to solve a specific problem. For example, a neural network "model" may refer to the entire system implemented as a neural network that has problem-solving ability through training. In this case, the neural network may have problem-solving ability by optimizing parameters connecting nodes or neurons through training. The neural network "model" may include a single neural network or may include a neural network set that combines a plurality of neural networks.

The description of the above-described terms is intended to help understanding of the present disclosure. Therefore, when the above-described terms are not explicitly described as matters limiting the contents of the present disclosure, it should be noted that they are not used in a sense limiting the technical ideas of the contents of the present disclosure.

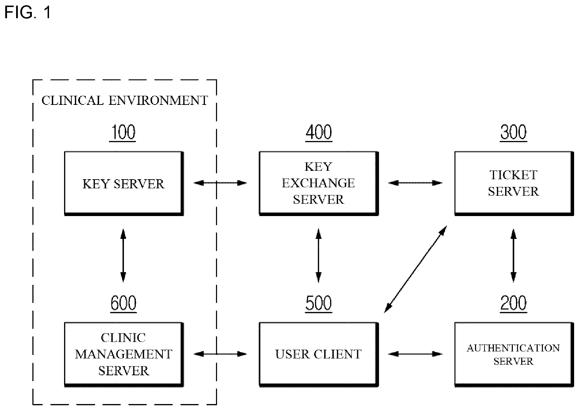

FIG. 1 is a block diagram illustrating a system for de-identification of personal information in medical services according to an embodiment of the present disclosure.

A system according to one embodiment of the present disclosure may include a key server 100 that is located in a clinical environment and holds a key for encryption and decryption of medical data, an authentication server 200 that authenticates access information about a user client 500 and issues a token for maintaining a session, a ticket server 300 that issues a ticket by which a user client 500 is authorized to receive a key based on a request by the user client 500 for access to encrypted medical data, a key exchange server 400 that transmits a key issued from a key server to the user client 500 based on a request for decryption of the encrypted medical data of the user client 500, and a clinic management server 600 that is located in the clinical environment and manages the medical data. Meanwhile, the user client 500 may be a web server that provides medical services.

When a server or client located in the clinical environment requests the key for encryption of the medical data, the key server 100 may provide the key to the corresponding server or client. In addition, when the key exchange server 400 requests the key for decryption of medical data, the key server 100 may provide the key to the key exchange server 400 that requested the key. In other words, when the server or client accessing the key server 100 is not located within the clinical environment, the key server 100 may not provide the key to servers or clients rather than to the key exchange server 400. Accordingly, when the key exchange server 400 requests a key, the key server 100 may form a communication trust interval so that only the key exchange server 400 may access the key server 100 and provide the key to the key exchange server 400. For example, when the key exchange server 400 requests a key, the key server 100 may block external communication and communicate only with the key exchange server 400 through a method such as virtual private network (VPN) tunneling.

When the access to the encrypted medical data is performed through the user client 500, the authentication server 200 may authenticate the access information of the user client 500 and issue a token to the user client 500 for maintaining the communication session with the user client 500. For example, when a user logs in to the medical service through the user client 500, the authentication server 200 may verify whether a normal user has attempted to log in based on the login information of the user client 500. When the authentication of the login information is completed and thus it is confirmed that the access is from a normal user, the authentication server 200 may issue a token and provide the token to the user client 500 so that the user client 500 may maintain the access state of the medical service.

The ticket server 300 may generate a ticket by which the user client 500 is authorized to receive the key from the key server 100 during the decryption process of the medical data. The ticket server 300 may transfer the generated ticket to the user client 500. In this case, the user client 500 that may receive the generated ticket through the ticket server 300 may be a client whose token received from the authentication server 200 has been verified. That is, the ticket server 300 may perform the verification of the token held by the user client 500 through communication with the authentication server 200, and then issue a ticket to the client whose token has been verified.

The key exchange server 400 may perform the role of requesting a key for decrypting medical data from the key server 100 and transmitting the key to the user client 500. For example, when the user client 500 requests a key from the key exchange server 400, the key exchange server 400 may request a ticket issuance while receiving the token transmitted from the user client 500 and transmitting the token to the ticket server 300. When the ticket server 300 performs the verification of the token through communication with the authentication server 200 and transmits the ticket issued to the user client 500 to the key exchange server 400, the key exchange server 400 may request a key from the key server 100 and receive the key. The key exchange server 400 may transmit the key received from the key server 100 to the user client 500. In this case, the key exchange server 400 may encrypt the key received from the key server 100 using the ticket and transmit the encrypted key to the user client 500.

The clinic management server 600 may receive medical data through communication with a device generating medical data or a web server and encrypt the received data. In this case, the clinic management server 600 may receive a key through communication with the key server 100 and encrypt a portion corresponding to personal information in the medical data. Since the clinic management server 600 is located within the clinical environment, the clinic management server 600 may freely receive a key through communication with the key server 100.

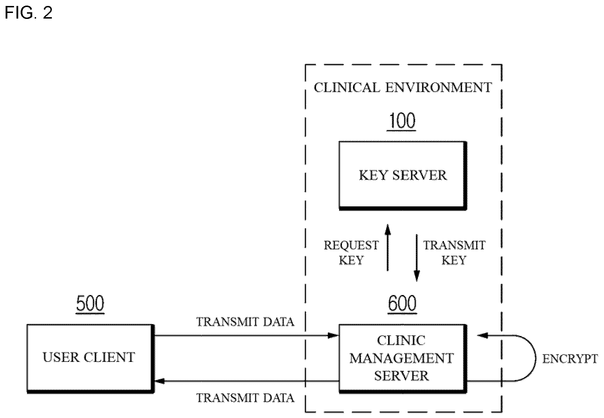

FIG. 2 is a block diagram illustrating an encryption process according to an embodiment of the present disclosure.

Referring to FIG. 2, the clinic management server 600 may receive medical data, such as an electrocardiogram, from the user client 500 including the device generating the medical data or the web server. Upon receiving the medical data, the clinic management server 600 may request a key from the key server 100 for encryption of the medical data. Since the clinic management server 600 is located within the clinical environment, the key server 100 may transmit the key to the clinic management server 600. The clinic management server 600 may encrypt the medical data using the key transmitted from the key server 100. The clinic management server 600 may upload the encrypted data to the user client 500 corresponding to the web server.

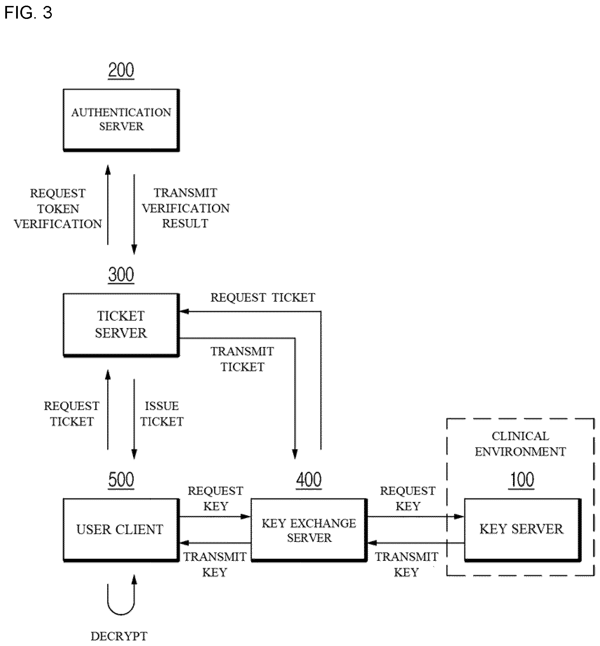

FIG. 3 is a block diagram illustrating a decryption process according to an embodiment of the present disclosure.

Referring to FIG. 3, when the ticket server 300 receives an access request including a token issued by the authentication server 200 from the user client 500, the ticket server 300 may verify the token through communication with the authentication server 200. When the token verification is completed by the authentication server 200, the ticket server 300 may issue a ticket to the user client 500. Specifically, the user client 500 may transmit the token issued by the authentication server 200 and a first timestamp to the ticket server 300. The ticket server 300 may verify the validity of the first timestamp. When the first timestamp is valid, the ticket server 300 may transmit the token transmitted from the user client 500 and a second timestamp to the authentication server 200. The authentication server 200 may verify the validity of the second timestamp. When the second timestamp is valid, the authentication server 200 may verify the token transmitted from the ticket server 300 and transmit the verification result to the ticket server 300. When the token verification is completed by the authentication server 200, the ticket server 300 may issue a ticket to the user client 500.

When the key exchange server 400 receives a decryption request including the token issued by the authentication server 200 from the user client 500 that is the web server, the key exchange server 400 may transmit the token to the ticket server 300 to request the ticket issuance. The ticket server 300 that the key exchange server 400 requests to issue a ticket may verify the token transmitted from the key exchange server 400 through communication with the authentication server 200. When the token verification by the authentication server 200 is completed, the ticket server 300 may transmit a ticket issued to the user client 500 based on the token to the key exchange server 400. When the key exchange server 400 receives a ticket issued to the user client 500 based on the token from the ticket server 300, the key exchange server 400 may receive a key for decryption of the encrypted medical data through communication with the key server 100. The key exchange server 400 may encrypt a key received through communication with the key server 100 with a ticket issued to the user client 500. The key exchange server 400 may transmit the key encrypted with the ticket to the user client 500. The user client 500 may decrypt the key received from the key exchange server 400 using the ticket issued through the ticket server 300. The user client 500 may decrypt the medical data using the decrypted key through the ticket. **In** this case, the user client 500 may not store the key received from the key exchange server 400, and use the key for decryption of the medical data and then immediately discards the key.

Specifically, the user client 500 may transmit the token issued through the authentication server 200 and a third timestamp to the key exchange server 400. The key exchange server 400 may verify the validity of the third timestamp. When the third timestamp is valid, the key exchange server 400 may transmit the token transmitted from the user client 500 and a fourth timestamp to the ticket server 300. The ticket server 400 may confirm the validity of the fourth timestamp. When the fourth timestamp is valid, the ticket server 300 may transmit the token transmitted from the key exchange server 400 and a fifth timestamp to the authentication server 200. The authentication server 200 may verify the validity of the fifth timestamp. When the fifth timestamp is valid, the authentication server 200 may verify the token transmitted from the ticket server 300 and transmit the verification result to the ticket server 300. When the verification of the token is completed by the authentication server 200, the ticket server 300 may transmit the ticket already issued to the user client 500 to the key exchange server 400. The key exchange server 400 may request a key from the key server 100. The key server 100 may form a communication trust interval and transmit the key to the key exchange server 400 located outside of a clinical environment. The key exchange server 400 may encrypt the key transmitted from the key server 100 using the ticket transmitted from the ticket server 300. The key exchange server 400 may transmit the key encrypted with the ticket to the user client 500. The user client 500 may decrypt the key transmitted from the key exchange server 400 with the ticket. The user client 500 may decrypt the medical data using the decrypted key through the ticket.

Meanwhile, the validity of the timestamp transmitted in the above-described decryption process may be understood with regard to a task of determining whether the timestamp is within a preset validity period. In other words, when the timestamp is determined to have exceeded the validity period through the validity verification, each server may discard the received information corresponding to the timestamp that has exceeded the validity period. For example, when the timestamp has exceeded a validity period of 5 seconds, each server may determine that the information corresponding to the timestamp is invalid and discard it.

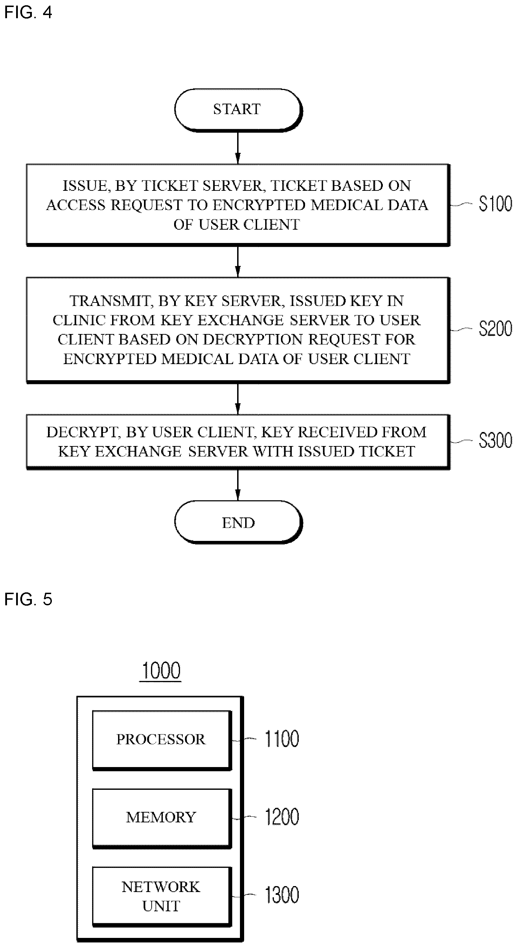

FIG. 4 is a flowchart illustrating a method of de-identification of personal information in medical services according to an embodiment of the present disclosure.

Referring to FIG. 4, based on the request by the user client for access to the encrypted medical data, the ticket server may issue a ticket (S100). In this case, the access request may include a token previously issued by the user client through the authentication server for access to the medical service. Upon receiving the access request including the token, the ticket server may verify the token through communication with the authentication server. When the token is verified by the authentication server, the ticket server may issue the ticket to the user client.

The key exchange server may transmit the key issued from the key server within the clinic to the user client based on the request by the user client for decryption of the encrypted medical data (S200). In this case, the access request may include the token previously issued by the user client through the authentication server for access to the medical service. Upon receiving the decryption request including the token, the key exchange server may transmit the token to the ticket server to request the ticket issuance. The ticket server may verify the token through communication with the authentication server. When the token is verified, the ticket server may transmit the ticket to the key exchange server. In this case, the ticket may be the ticket issued to the user client through operation S100. The key exchange server may receive the key through communication with the key server and encrypt the key using the ticket. The key exchange server may transmit the encrypted key to the user client.

The ticket issued by the user client may decrypt the key received from the key exchange server (S300). Since the key received from the key exchange server is encrypted via the ticket, the user client may decrypt the key using the issued ticket. The user client may decrypt the medical data using the decrypted key.

FIG. 5 is a block diagram of a computing device constituting the system according to an embodiment of the present disclosure.

A computing device 1000 according to an embodiment of the present disclosure may be a hardware device or a part of a hardware device that performs comprehensive processing and calculation of data, or may be a software-based computing environment connected to a communication network. For example, the computing device 1000 may be a server that performs intensive data processing functions and shares resources within a system for de-identification of personal information in medical services.

Referring to FIG. 5, the computing device 1000 according to an embodiment of the present disclosure may include a processor 1100, a memory 1200, and a network unit 1300. However, FIG. 1 is only one example, and the computing device 1000 may include other configurations for implementing a computing environment. In addition, only some of the configurations disclosed above may be included in the computing device 1000.

The processor 1100 according to an embodiment of the present disclosure may be understood as a configuration unit including hardware and/or software for performing computing operations. For example, the processor 1100 may read a computer program and perform data processing for machine learning. The processor 1100 may process computational processes such as processing input data for machine learning, feature extraction for machine learning, and error calculation based on backpropagation. The processor 1100 for performing such data processing may include a central processing unit (CPU), a general purpose graphics processing unit (GPGPU), a tensor processing unit (TPU), an application specific integrated circuit (ASIC), or a field programmable gate array (FPGA), etc. The type of the processor 1100 described above is only one example, and thus the type of the processor 1100 may be configured in various ways within a category understandable to those skilled in the art based on the contents of the present disclosure.

The memory 1200 according to an embodiment of the present disclosure may be understood as a configuration unit including hardware and/or software for storing and managing data processed in the computing device 1000. That is, the memory 1200 may store any type of data generated or determined by the processor 1100 and any type of data received by the network unit 1300. For example, the memory 1200 may include at least one of storage media such as a flash memory type, a hard disk type, a multimedia card micro type, a card type memory (for example, an SD or XD memory, or the like), a random access memory (RAM), a static random access memory (SRAM), a read-only memory (ROM), an electrically erasable programmable read-only memory (EEPROM), a programmable read-only memory (PROM), a magnetic memory, a magnetic disk, and an optical disc. In addition, the memory 1200 may include a database system that controls and manages data in a predetermined system. Since the type of the memory 1200 described above is only one example, the type of the memory 1200 may be configured in various ways within a category understandable to those skilled in the art based on the contents of the present disclosure.

The network unit 1300 according to an embodiment of the present disclosure may be understood as a configuration unit that transmits and receives data through any known wired/wireless communication system. For example, the network unit 1300 may transmit and receive data using wired or wireless communication systems such as a local area network (LAN), Wideband Code Division Multiple Access (WCDMA), Long Term Evolution (LTE), wireless broadband Internet (WiBro), 5th generation mobile communication (5G), ultra wide-band, ZigBee, radio frequency (RF) communication, wireless LAN, wireless fidelity (WIFI), near field communication (NFC), or Bluetooth. Sine the above-described communication systems are only examples, the wired and wireless communication system for data transmission and reception of the network unit 1300 may be applied in various ways other than the above-described examples.

Various embodiments of the present disclosure described above may be combined with additional embodiments and can be modified within a range that may be understood by those skilled in the art in light of the detailed description set forth above. The embodiments of the present disclosure are illustrative in all respects and should be understood as non-limiting. For example, each component described as a single type may be implemented in a distributed manner, and similarly, components described as distributed may be implemented in a combined form. Accordingly, all changes or modifications derived from the meaning, scope, and equivalent concept of the claims of the present disclosure should be construed as being included in the scope of the present disclosure.

## Claims

1. A system for de-identification of personal information in medical services, comprising:
a key server that has a key for encryption and decryption of medical data in a clinic environment;
an authentication server that authenticates access information about a user client and issues a token for maintaining a session;
a ticket server that issues, based on a request by a user client for access to the encrypted medical data, a ticket by which the user client is authorized to receive the key; and
a key exchange server that transmits, to the user client, based on a request by the user client for decryption of the encrypted medical data, the key issued by the key server.

2. The system of claim 1, wherein the key server forms a communication trust interval so that only the key exchange server accesses the key server, when the server or client requesting the key is not located within the clinic environment.

3. The system of claim 1, wherein, when the ticket server receives, from the user client, an access request including the token issued by the authentication server,
the ticket server verifies the token through communication with the authentication server, and when the token verification is completed by the authentication server, issues the ticket to the user client.

4. The system of claim 1, wherein, when the key exchange server receives, from the user client, the decryption request including the token issued by the authentication server,
the key exchange server transmits the token to the ticket server to request the issuance of the ticket, and
the ticket server verifies the token transmitted from the key exchange server through the communication with the authentication server, and when the token verification is completed by the authentication server, transmits the ticket issued to the user client to the key exchange server based on the token.

5. The system of claim 4, wherein, when the key exchange server receives, from the ticket server, the ticket issued to the user client based on the token,
the key exchange server receives a key for decryption of the encrypted medical data through the communication with the key server, encrypts the key received through the communication with the key server with the ticket that issues the token to the user client, and transmits the key encrypted with the ticket to the user client.

6. The system of claim 5, wherein the user client decrypts the key received from the key exchange server using the ticket issued through the ticket server, and
decrypts the medical data using the key decrypted through the ticket.

7. The system of claim 6, wherein the user client does not store the key received from the key exchange server, and uses the key for the decryption of the medical data and then discards the key.

8. The system of claim 1, wherein the authentication server, the ticket server, or the key exchange server performs validity verification on a timestamp generated during the communication with the user client or communication between servers, and
discards the received information corresponding to the timestamp whose validity period has been exceeded when the timestamp is determined to have exceeded its validity period through the validity verification.

9. A method of de-identification of personal information in medical services, comprising:
Issuing, by a ticket server, a ticket based on a request by a user client for access to encrypted medical data;
transmitting, by a key exchange server, a key issued from a key server in a clinic to the user client based on a request by the user client for decryption of the encrypted medical data; and
decrypting, by the user client, the key transmitted from the key exchange server using the issued ticket.
